# EUROPÄISCHE PATENTSCHRIFT

(11) **EP 3 768 346 B1**
(45) Veröffentlichungstag und Bekanntmachung des Hinweises auf die Patenterteilung: **18.03.2026**
(21) Anmeldenummer: 19705467.9
(22) Anmeldetag: 06.02.2019
(51) Int. Cl.: A61M 60/135, A61M 60/857, A61M 60/13, A61M 60/165, A61M 60/295, A61M 60/497, A61M 60/562, A61M 60/585, A61M 60/894

(54) **PUMPKATHETER ZUM GERICHTETEN PULSATILEN LEITEN VON BLUT**
PUMP CATHETER FOR THE DIRECTED PULSATILE CONVEYING OF BLOOD
CATHÉTER DE POMPE POUR LA CONDUITE PULSATILE DIRIGÉE DU SANG

(30) Priorität: 14.02.2018 DE 102018103364
(43) Veröffentlichungstag der Anmeldung: 27.01.2021
(73) Patentinhaber: Novapump GmbH, 07749 Jena (DE)
(72) Erfinder: PATZER, Patrick, 07570 Harth-Pöllnitz (DE); PFEIFER, Jörg, 07743 Jena (DE); REICH, Ronald, 07749 Jena (DE)
(74) Vertreter: Carlsohn, Marc René
(86) Internationale Anmeldenummer: PCT/EP2019/052953
(87) Internationale Veröffentlichungsnummer: WO 2019/158420

(56) Entgegenhaltungen:
- WO-A1-00/35515
- WO-A1-2019/079342
- US-A- 5 891 012

## Beschreibung

### Beschreibung der Erfindung

Die vorliegende Erfindung betrifft einen Pumpkatheter zum gerichteten pulsatilen Leiten von Blut gemäß dem Oberbegriff von Anspruch 1.

Katheter der eingangs genannten Art sind als Stand der Technik beispielsweise aus WO 2008/113785 A2, DE 10 2014 003 153 A1, DE 10 2014 012 850 A1 und US5891012 A bekannt. Solche Katheter werden vorzugsweise zur temporären Herz- und Kreislaufunterstützung bei akut eingeschränkter Herzleistung bzw. Herzinsuffizienz mit Hilfe etablierter Kathetertechnik perkutan - z.B. über eine Öffnung des Leistengefäßes - im menschlichen Körper platziert. Insbesondere kann er auch bei einer höhergradigen Aortenklappeninsuffizienz eingesetzt werden. Er dient dazu, die zu leitende Körperflüssigkeit von einem ersten Ort an einen anderen Ort zu transportieren, ohne den Druck der Flüssigkeit am ersten Ort signifikant über den physiologisch vorgegebenen Zustand hinaus zu erhöhen, indem er das Prinzip einer Tauchpumpe realisiert und vorzugsweise durch Einsatz eines Ballonkatheters mit dem Prinzip einer Membranpumpe kombiniert.

Eine kritische Größe solcher gattungsgemäßen Leitungskatheter ist die Menge an Blut, die pro Zeiteinheit durch den Katheter geleitet werden kann. Diese sollte möglichst groß sein, bei gleichzeitig möglichst geringer Belastung der empfindlichen Blutbestandteile.

Der Erfindung liegt daher die Aufgabe zugrunde, einen Katheter der eingangs genannten Art anzugeben, der gegenüber vorbekannten Pumpkathetern ein verbessertes Design aufweist.

Diese Aufgabe wird durch einen Katheter gelöst, welcher die in Anspruch 1 angegebenen Merkmale aufweist. In den Unteransprüchen sind vorteilhafte Ausgestaltungen angegeben.

Die Erfindung sieht vor, dass ein proximaler Abschnitt und ein distaler Abschnitt, der eine expandierbare Pumpkammer und einen distal von der Pumpkammer angeordneten Schlauchabschnitt mit einer Auslassöffnung umfasst, vorhanden sind, und ist dadurch gekennzeichnet, dass zwischen dem proximalen Abschnitt und der Pumpkammer ein Einlasselement mit mindestens einer Einlassöffnung angeordnet ist und das Einlasselement und die Pumpkammer durch ein Koppelelement voneinander geometrisch beabstandet sind.

"Distal" bedeutet im Sinne der Erfindung "zu dem in den Körper eingeschobenen Ende des Katheters hin". "Proximal" bedeutet im Sinne der Erfindung "vom distalen Katheterende weg". Bei dem erfindungsgemäßen Katheter ist also ein proximales Katheterende dem distalen Katheterende gegenüberliegend angeordnet, und ragt, wenn der Katheter bestimmungsgemäß in den Körper eingesetzt ist, in der Regel aus diesem heraus.

Durch den mehrteiligen Aufbau des Katheters mit einem von der Pumpkammer räumlich beabstandeten Einlasselement werden strömungsdynamische Vorteile gegenüber den heute bekannten Lösungen realisiert. Die durch bei einem pulsatilen Anschub anormal viskoser Flüssigkeiten wie z.B. menschlichem Blut auftretenden Scherkräfte werden durch den erfindungsgemäßen Aufbau minimiert und es wird eine laminare und beruhigte Strömung im Katheterinneren ermöglicht. Das zwischen der Pumpkammer und dem Einlasselement angeordnete Koppelelement bildet dabei eine strömungswirksame Größe.

Anders als bei vorbekannten Pumpkatheterkonzepten, die Einlassöffnungen im Bereich der Pumpkammer vorsehen, führt die geometrische Trennung der Einlassöffnungen von der Pumpkammer dazu, dass das Einlasselement unabhängig von der Pumpkammer anatomisch günstig gestaltet werden kann, womit die Blutaufnahme in den Katheter im Vergleich zu Einlassöffnungen, die an der Außenwand der Pumpkammer verteilt angeordnet sind, deutlich verbessert wird.

Der möglichst schonende Transport des Blutes ist wichtig. Mit der Erfindung können heutige Nachteile perkutan platzierbarer temporärer Herzpumpen wie z.B. erhöhte Hämolyse-Raten und die damit einhergehende Neigung zur Bildung von Thrombozyten minimiert werden. Weiterhin werden Reibungsverluste minimiert. Dies hat Vorteile bezüglich einer erhöhten pulsatilen Pumpleistung.

Der proximale Abschnitt des Katheters dient der Manipulation und der korrekten Platzierung des distalen funktionalen Abschnitts im Patienten. Der proximale Abschnitt ist schlauchförmig ausgebildet. Bevorzugt verlaufen durch das Schlauchlumen des proximalen Teils Versorgungsleitungen, die mit Strukturen des distalen Teils kommunizieren. Der proximale Abschnitt ist einerseits ausreichend flexibel, um sich bei der Implantation des Katheters in einen Patienten weitgehend der Patientenanatomie anzupassen und andererseits ausreichend rigide, um einen Vorschub des distalen Abschnitts an seinen Bestimmungsort innerhalb des Patienten zu ermöglichen.

Der distale Abschnitt einschließlich des Einlasselements wirkt als Blutpumpe und ist eingerichtet, Blut von einem ersten Ort zu einem zweiten Ort zu leiten. Bevorzugt ist der Katheter zur Unterstützung der Pumpleistung des rechten Herzens vorgesehen. In diesem Fall wird der Katheter mit dem distalen Abschnitt voran über einen Zugang in der Leiste in den Patientenkörper bis zum rechten Herzen vorgeschoben bis zu seiner bestimmungsgemäßen Lage im Patientenkörper. In dieser Lage durchspannt der distale Katheterabschnitt das Rechtsherz vollständig. Bestimmungsgemäß sollen das Einlasselement mit der Einlassöffnung und die Pumpkammer im Bereich der unteren Hohlvene *(Vena cava inferior),* d.h. in Flussrichtung des Blutes vor dem rechten Ventrikel, zu liegen kommen. Der distal von der Pumpkammer angeordnete Schlauchabschnitt soll den rechten Ventrikel durchspannen, so dass die Auslassöffnung idealerweise im Bereich der Pulmonalarterie angeordnet ist. In dieser Soll-Position kann im Bereich der Hohlvene über die Einlassöffnung Blut in das Katheterinnere gesaugt werden. Anschließend kann das in das Katheterinnere gesaugte Blut in distale Richtung gepumpt werden (d.h. durch die Pumpkammer und den distalen Schlauchabschnitt hindurch), um den Katheter im Bereich der Pulmonalarterie (Lungenarterie, *Aorta pulmonaris)* über die distale Auslassöffnung wieder zu verlassen.

Innerhalb der Pumpkammer ist im für den Einsatz am Patienten konfigurierten Zustand des Katheters ein mit einem Fluid befüllbarer Ballon angeordnet. Aufgrund einer verdrängenden Wirkung des befüllten Ballons wird ein gerichteter Transport des Blutes durch das Katheterinnere in distale Richtung ermöglicht. Der Ballon wird über eine Zuleitung, die durch das Schlauchlumen des proximalen Teils geführt ist, von außerhalb des Katheters mit dem Fluid (in der Regel ein Gas wie beispielsweise Helium) versorgt. Die Zuleitung endet am proximalen Katheterende oder reicht aus diesem heraus. Die Zuleitung bzw. das proximale Katheterende kann an eine (extrakorporale) Pumpeinheit angeschlossen sein. Die Pumpeinheit kann mittels einer (integrierten) Steuereinheit so gesteuert sein, dass der Ballon periodisch mit dem Fluid befüllt wird. Die Einheit aus Ballon und Druckschlauch kann durch einen im Stand der Technik bekannten intraaortalen Ballonkatheter (IAB-Katheter) gebildet sein, der herkömmlich bei dem sogenannten intraaortalen Gegenpulsationsverfahren zum Einsatz kommt. Die Pumpeinheit kann durch eine im Stand der Technik bekannte IABP-Konsole (engl. IABP = Intra Aortic Balloon Pump) gebildet sein. Eine solche Konsole ist typischerweise hinsichtlich der Frequenz der Befüllvorgänge des Ballons mit Fluid und/oder des Volumens an Fluid pro Befüllvorgang einstellbar.

Vorzugsweise ist das Einlasselement expandierbar. Die Eigenschaft "expandierbar" bedeutet im Sinne der Erfindung, dass das Einlasselement zwischen zwei Konfigurationen mit unterschiedlichen Innenvolumen umschaltbar ist. Die Konfiguration mit größerem Innenvolumen kann als "expandiert" bezeichnet werden, die andere Konfiguration kann als "gefaltet" bezeichnet werden. Insbesondere kann das Einlasselement einen selbstexpandierbaren, d.h. selbstaufstellenden, Stent umfassen.

An der Einlassöffnung und/oder im Bereich des Koppelabschnitts kann ein Rückschlagventil angeordnet sein. Das Rückschlagventil kann insbesondere als Folienventil ausgebildet sein.

Das Einlasselement kann eine Vielzahl von Einlassöffnungen umfassen.

Das Koppelelement kann eingerichtet sein, eine beim Implantieren und/oder Explantieren des Katheters auf den distalen Abschnitt einwirkende Kraft zumindest anteilig auf den proximalen Abschnitt zu übertragen. Insbesondere können das Einlasselement und/oder die Pumpkammer Stützstrukturen ausprägen, die sich in das Koppelelement erstrecken und dieses mechanisch stabilisieren. Bevorzugt sind die Stützstrukturen Erweiterungen des selbstaufstellenden Stents und ragen stegförmig in das Koppelelement. Bevorzugt umfasst das Koppelelement Koppelstrukturen, die formschlüssig und/oder kraftschlüssig an den Stützstrukturen des Einlasselements und/oder an den Stützstrukturen der Pumpkammer angreifen und die Kraftübertragung zumindest anteilig bewirken. Bevorzugt sind die Koppelstrukturen aus einem nichtmetallischen Material, insbesondere einem Polymer, hergestellt.

Bevorzugt ist die geometrische Ausführung des Einlasselements in der expandierten und in der gefalteten Konfiguration auf eine (individuelle) Patientengröße abgestimmt.

Nachfolgend wird die Erfindung anhand von Zeichnungen noch näher erläutert. Darin zeigt
Fig. 1 einen Ausschnitt eines Pumpkatheters mit einem Einlasselement mit mehreren Einlassöffnungen und einem Koppelelement,
Fig. 2 einen Ausschnitt eines Pumpkatheters mit einem Einlasselement mit mehreren Einlassöffnungen und Stützstrukturen, die sich in das Koppelelement erstrecken,
Fig. 3 einen Ausschnitt eines Pumpkatheters mit einem Einlasselement mit mehreren Einlassöffnungen und Stützstrukturen, die sich in das Koppelelement erstrecken sowie Koppelstrukturen im Bereich des Koppelelements, sowie
Fig. 4 einen Ausschnitt eines Pumpkatheters mit einem Einlasselement mit mehreren Einlassöffnungen und einem Koppelelement, das ein Rückschlagventil aufweist.

Übereinstimmende Teile tragen in den unterschiedlichen Figuren identische Bezugszeichen.

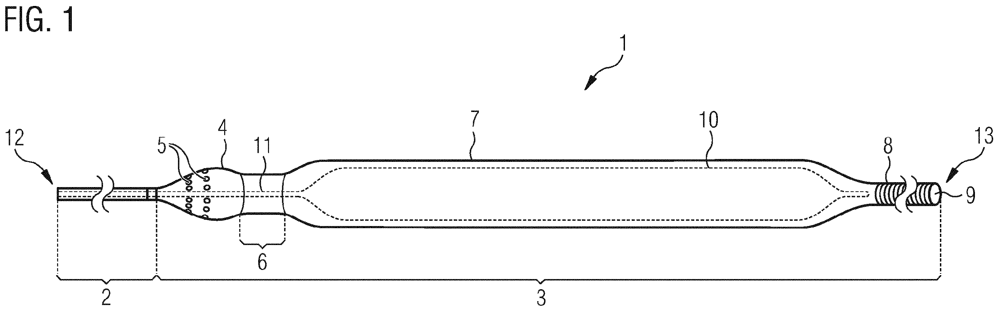

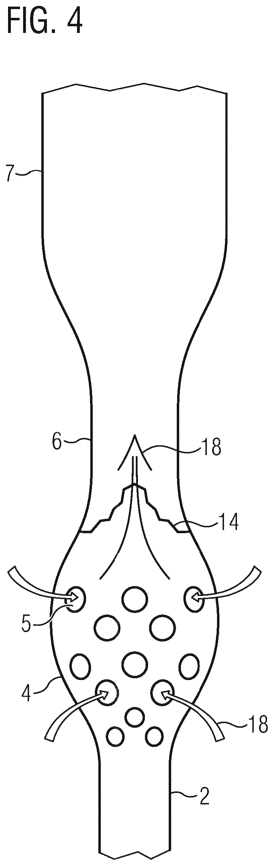

Fig. 1 zeigt in einer schematischen Darstellung ausschnittweise einen erfindungsgemäßen Pumpkatheter 1. An einen schlauchförmigen proximalen Abschnitt 2, der in Abbildung 1 nur angedeutet ist, grenzt ein distaler Abschnitt 3 an. Der distale Abschnitt 3 umfasst ein Einlasselement 4 mit mehreren Einlassöffnungen 5, ein Koppelelement 6, eine expandierbare Pumpkammer 7 und einen distal von der Pumpkammer 7 angeordneten Schlauchabschnitt 8 mit einer distalen Auslassöffnung 9 am distalen Katheterende 13. Der Schlauchabschnitt 8 ist wie der proximale Abschnitt 2 aus Gründen der Übersichtlichkeit ebenfalls nur angedeutet. Innerhalb der Pumpkammer 7 ist ein Ballon 10 angeordnet (gestrichelte Linien). An den Ballon 10 ist eine Zuleitung 11 (ebenfalls gestrichelt dargestellt) angeschlossen, die den Ballon 10 mit Helium versorgt und innerhalb des Katheters 1 bis zu dem proximalen Katheterende 12 verläuft. Der Katheter 1 kann vorzugsweise als Rechtsherzpumpkatheter verwendet werden. Für eine Anwendung am rechten Herzen wird der Katheter 1 über eine Öffnung der Körpervene in der Leistengegend mit dem distalen Ende 13 voran in den Patientenkörper vorgeschoben, und zwar so, dass der distale Schlauchabschnitt 8 das Rechtsherz durchspannt und damit überbrückt. Die Pumpkammer 7 soll idealerweise im Bereich der unteren Hohlvene, in Fließrichtung des Blutes vor dem rechten Vorhof des Herzens, platziert werden. Eine (teilweise) Platzierung der Pumpkammer 7 innerhalb des rechten Vorhofs ist alternativ möglich. Die Zuleitung 11 wird extrakorporal an eine Pumpe oder Pumpkonsole (nicht gezeigt) angeschlossen, die alternierend mit einer vorbestimmten Frequenz, z.B. mit etwa 100 bpm *(beats per minute)* Helium in den Ballon 10 pumpt und wieder aus ihm abzieht. Der Ballon 10 wirkt auf diese Weise nach dem Verdrängerprinzip als Antrieb für einen gerichteten Transport des den Katheter umgebenden Bluts in den Katheter 1 hinein und in distale Richtung durch den Katheter 1 hindurch bis zur Auslassöffnung 9. Die Pumpkammer 7, das Koppelelement 6 und das Einlasselement 4 sind ausreichend steif, um dem Sog beziehungsweise der Unterdruckwirkung des gerade entleerten inneren Ballons 10 zu widerstehen. Das Einlasselement 4 ist von der Pumpkammer 7 beabstandet angeordnet und weist eine Mehrzahl von Einlassöffnungen 5 auf. Der durch den entleerten Ballon 10 im Katheterinneren erzeugte Unterdruck führt dazu, dass den Katheter 1 im Bereich des Einlasselements 4 umgebendes Blut durch die Einlassöffnungen 5 in das Katheterinnere gesaugt wird und nach distal gerichtet in die Pumpkammer 7 strömt. Das Einlasselement 4 weist einen im Vergleich zur Pumpkammer 7 geringeren Außendurchmesser auf, so dass venöses Blut ungehindert zwischen der Gefäßwand und der Außenwand des Einlasselements 4 zirkulieren kann und sichergestellt ist, dass die Einlassöffnungen während der Ansaugphase frei zugänglich sind und nicht von der Wand des Blutgefäßes abgedichtet werden. Das Koppelelement 6 hat eine ungefähre Länge von 1 bis 2 cm. Dies reicht aus, um den Blutstrom in Richtung Pumpkammer signifikant zu beruhigen. In der Aufpumpphase des Ballons 10 wird das in den Katheter gesaugte Blut, bedingt durch den Druckanstieg im Inneren des Katheters 1, verdrängt. Um eine gerichtete Verdrängung optimal in distale Richtung zu ermöglichen, ist proximal von der Pumpkammer 7 ein Rückschlagventil 14 (vgl. Fig. 4) vorgesehen, das sich infolge des Druckanstiegs schließt und die Einlassöffnungen sperrt, sodass ein Rückfluss des Blutes aus den Einlassöffnungen hinaus ausgeschlossen wird. Bei anderen Ausführungsbeispielen kann auch an jeder einzelnen Einlassöffnung ein Rückschlagventil vorgesehen sein.

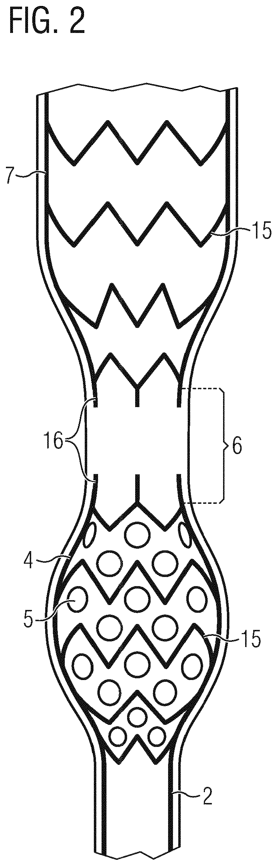

Bei dem erfindungsgemäßen Pumpkatheters (Fig. 2) sind im Bereich des Koppelelements 6 Stützstrukturen 16 vorgesehen, die von dem Einlasselement 4 und von der Pumpkammer 7 ausgehend in das Koppelelement 6 hineinragen und auf diese Weise das Koppelelement 6 mechanisch stabilisieren. Der Rahmen 15 für das Einlasselement 4 ist (ebenso wie der Rahmen 15 für die Pumpkammer 7) bevorzugt aus einer Hülse einer Formgedächtnislegierung, beispielsweise Nitinol, lasergeschnitten. Die geometrische Z-Struktur des Rahmens 15 in Verbindung mit dem Formgedächtnis-Verhalten des Rahmenmaterials bewirkt, dass der Rahmen 15 einerseits zuverlässig gefaltet und so für ein besseres Handling bei der Implantation und Explantation des Katheters 1 in den Patientenkörper bzw. aus dem Patientenkörper gegen einen Aufstelldruck - beispielsweise durch Überstreifen einer Hülse - im Durchmesser verkleinert werden kann und andererseits im aufgestellten Zustand ausreichend steif ist, dass das Einlasselement 4 und die Pumpkammer 7 bis zu einer vorbestimmten maximalen Druckdifferenz zwischen dem Innendruck und dem Umgebungsdruck nicht kollabieren.

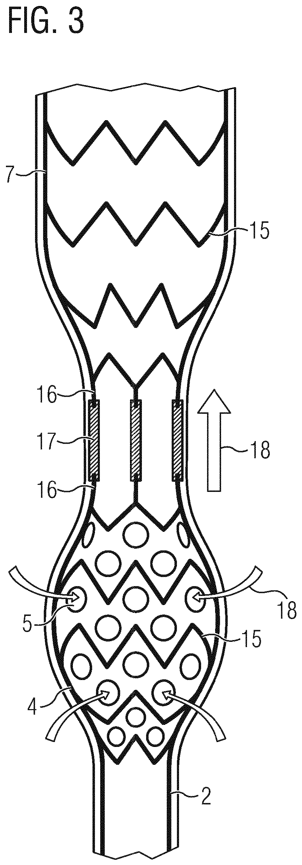

Außen ist der Rahmen 15 des Einlasselements 4 mit einer Hüllfolie bespannt. In die Hüllfolie des Einlasselements 4 sind Einlassöffnungen 5 gefertigt. Die Einlassöffnungen 5 können bei weiteren Ausführungsbeispielen mit Ventilen versehen sein. Dazu sind bei einigen Ausführungsbeispielen zwischen Hüllfolie und Rahmen 15 Folienstreifen vorgesehen. Die Folienstreifen sind ungefähr so breit wie eine einzelne Einlassöffnung 5 und so zwischen Hüllfolie und Rahmen 15 gespannt, dass die Einlassöffnungen 5 gerade verdeckt sind. Die Folienstreifen sind punktuell oder entlang von Linien quer zum Folienstreifen mit der Hüllfolie verbunden. Wichtig ist, dass Bereiche des Folienstreifens nicht mit der Hüllfolie verbunden sind. Bei einem Überdruck innerhalb des Einlasselements 4 wird der Folienstreifen von innen an die Hüllfolie gepresst und sperrt beziehungsweise dichtet auf diese Weise die Einlassöffnungen 5 ab. Ein Unterdruck im Inneren des Einlasselements 4 bewirkt dagegen, dass umgebendes Blut durch die Einlassöffnungen 5 gegen den Folienstreifen drückt. Dies hat zur Folge, dass der Folienstreifen nach innen von der Hüllfolie abhebt, sodass die Sperrwirkung an den Einlassöffnungen 5 aufgehoben ist. Dadurch, dass Bereiche des Streifens nicht mit der Hüllfolie verbunden sind, kann Blut in das Innere des Einlasselements 4 strömen (vgl. Blutstrom 18 bei Figuren 3, 4).

Das zwischen dem Einlasselement 4 und der Pumpkammer 7 angeordnete Koppelelement 6 sorgt für eine Beruhigung des Blutstromes 18 in distale Richtung, was einen insgesamt schonenderen Transport des Blutes durch den Katheter 1 ermöglicht. Das Koppelelement 6 ist wie schon erwähnt mittels der Stützstrukturen 16 ausgesteift. In einigen Ausführungsbeispielen sind die Stützstrukturen 16 Stege, die jeweils an den selbstaufstellenden Rahmen 15 ausgebildet sind (vgl. Figuren 2, 3). Außen sind die Stützstrukturen 16 in gleicher Weise wie der Rahmen 15 des Einlasselements 4 mit einer Hüllfolie bespannt. Wie das Einlasselement 4 kann auch das Koppelelement 6 für ein optimiertes Handling mit katheterbasierter Implantation und Explantation "gefaltet" beziehungsweise durch Überstreifen einer Hülse im Durchmesser verkleinert werden. Das Koppelelement 6 ist vorteilhaft zwischen 5 und 20 mm lang und weist (im aufgestellten Zustand) einen Innendurchmesser zwischen 5 und 15 mm auf.

Im Ausführungsbeispiel der Fig. 3 ist eine Weiterbildung des Koppelelements 6 des Katheters 1 der Fig. 2 gezeigt. Das Koppelelement 6 der Fig.3 weist zusätzlich Koppelstrukturen 17 auf. Die Koppelstrukturen 17 bewirken eine weiter verbesserte Aussteifung des Koppelelements 6. Die Koppelstrukturen 17 greifen an den Stützstrukturen 16 an. Bei einigen Ausführungsbeispielen sind die Koppelstrukturen 17 dünne Stäbe. Die Stäbe können beispielsweise aus einem körperverträglichen Kunststoff oder einem Metall sein. Sofern Vollstäbe verwendet werden, weisen diese beiderseits an ihren Stabenden Bohrungen auf, in die die Stützstrukturen 16 gesteckt, geklebt, geschraubt werden können. Analog funktionieren auch Hohlstäbe bzw. Hülsen. Auf diese Weise ist eine form- und/oder kraftschlüssige Verbindung hergestellt, was die Handhabung des Katheters 1 insbesondere bei der Implantation und Explantation weiter verbessert.

Im Ausführungsbeispiel der Fig. 4 schließlich weist das Koppelelement 6 des erfindungsgemäßen Katheters 1 zusätzlich ein Ventil 14 auf. Durch die Anordnung des Ventils 14 wird der gerichtete Transport des Blutes in distale Richtung verbessert: das Ventil 14 lässt Blut in Flussrichtung 18 passieren und sperrt den Blutfluss in die Gegenrichtung. Bei dem Ausführungsbeispiel der Fig. 4 ist das Ventil 14 durch einen Folienvorhang 14 gebildet, der radial mit der Hüllfolie des Koppelelements 6 verbunden (verklebt, verschweißt) ist und Blut in Richtung 18 passieren lässt, in Gegenrichtung aber nicht. Dazu kann in Flussrichtung 18 gesehen hinter dem Vorhang 14 ein Gitter (aus Übersichtlichkeitsgründen in Fig. 4 nicht dargestellt) platziert sein gegen das der Vorhang 14 gepresst wird, wenn Blut entgegen der Richtung 18 gegen den Vorhang 14 drückt. Das Gitter kann beispielsweise durch eine Lochfolie gebildet sein, die hinter dem Vorhang ebenfalls radial mit der Hüllfolie des Koppelelements 6 verbunden (verklebt, verschweißt) ist.

### Bezugszeichenliste

- 1: Pumpkatheter
- 2: proximaler Abschnitt
- 3: distaler Abschnitt
- 4: Einlasselement
- 5: Einlassöffnung / 5' Einlassventil
- 6: Koppelelement
- 7: Pumpkammer
- 8: distaler Schlauchabschnitt
- 9: Auslassöffnung
- 10: Ballon
- 11: Zuleitung
- 12: proximales Katheterende
- 13: distales Katheterende
- 14: Rückschlagventil
- 15: selbstaufstellender Rahmen
- 16: Stützstruktur
- 17: Koppelstruktur
- 18: Flussrichtung des Blutes

## Patentansprüche

1. Pumpkatheter (1) zum gerichteten pulsatilen Leiten von Blut, umfassend:
einen proximalen Abschnitt (2),
einen distalen Abschnitt (3), wobei der distale Abschnitt (3) eine expandierbare Pumpkammer (7) und einen distal von der Pumpkammer (7) angeordneten Schlauchabschnitt (8) mit einer Auslassöffnung (9) umfasst,
wobei zwischen dem proximalen Abschnitt (2) und der Pumpkammer (7) ein Einlasselement (4) mit mindestens einer Einlassöffnung (5) angeordnet ist
**dadurch gekennzeichnet dass**
das Einlasselement (4) und die Pumpkammer (7) durch ein Koppelelement (6) voneinander geometrisch beabstandet sind,
wobei das Koppelelement (6) eingerichtet ist, eine beim Implantieren und/oder Explantieren des Katheters (1) in einen Patientenkörper auf den distalen Abschnitt (3) einwirkende Kraft zumindest anteilig auf den proximalen Abschnitt (2) zu übertragen, indem das Einlasselement (4) und/oder die Pumpkammer (7) Stützstrukturen (16) ausprägen, die sich in das Koppelelement (6) erstrecken und dieses mechanisch stabilisieren.

2. Pumpkatheter nach Anspruch 1, **dadurch gekennzeichnet, dass** das Einlasselement (4) expandierbar ist.

3. Pumpkatheter nach Anspruch 1 oder 2, **dadurch gekennzeichnet, dass** an der Einlassöffnung (5) und/oder im Bereich des Koppelelements (6) ein Rückschlagventil (14) angeordnet ist.

4. Pumpkatheter nach einem der vorherigen Ansprüche, **dadurch gekennzeichnet, dass** das Einlasselement (4) eine Vielzahl von Einlassöffnungen (5) oder Einlassventilen (5') umfasst.

5. Pumpkatheter nach Anspruch 1, **dadurch gekennzeichnet, dass** das Koppelelement (6) Koppelstrukturen (17) umfasst, die formschlüssig und/oder kraftschlüssig an den Stützstrukturen (16) des Einlasselements (4) und/oder an den Stützstrukturen (16) der Pumpkammer (7) angreifen und die Kraftübertragung zumindest anteilig bewirken.

6. Pumpkatheter nach Anspruch 5, **dadurch gekennzeichnet, dass** die Koppelstrukturen (17) ein nichtmetallisches Material, bevorzugt ein Polymer, umfassen.

## Claims

1. Pump catheter (1) for the directed pulsatile conveying of blood, comprising:
a proximal portion (2),
a distal portion (3), the distal portion (3) comprising an expandable pump chamber (7) and a tube portion (8) which is arranged distally from the pump chamber (7) and has an outlet opening (9),
an inlet element (4) which has at least one inlet opening (5) being arranged between the proximal portion (2) and the pump chamber (7),
**characterized in that**
the inlet element (4) and the pump chamber (7) are geometrically spaced apart from one another by a coupling element (6),
the coupling element (6) being configured to transfer at least a portion of the force acting on the distal portion (3) during implantation and/or explantation of the catheter (1) into/from a patient's body to the proximal portion (2) **in that** the inlet element (4) and/or the pump chamber (7) form support structures (16) which extend into the coupling element (6) and mechanically stabilize it.

2. Pump catheter according to claim 1, **characterized in that** the inlet element (4) is expandable.

3. Pump catheter according to claim 1 or claim 2, **characterized in that** a check valve (14) is arranged at the inlet opening (5) and/or in the region of the coupling element (6).

4. Pump catheter according to any of the preceding claims,
**characterized in that** the inlet element (4) comprises a plurality of inlet openings (5) or inlet valves (5').

5. Pump catheter according to claim 1, **characterized in that** the coupling element (6) comprises coupling structures (17) which engage form-fittingly and/or frictionally on the support structures (16) of the inlet element (4) and/or on the support structures (16) of the pump chamber (7) and effect at least a portion of the force transfer.

6. Pump catheter according to claim 5, **characterized in that** the coupling structures (17) comprise a non-metal material, preferably a polymer.

## Revendications

1. Cathéter de pompage (1) pour l'acheminement pulsatile dirigé du sang, comprenant :
une section proximale (2),
une section distale (3), dans lequel la section distale (3) comprend une chambre de pompage (7) expansible et une section tubulaire (8) disposée de manière distale par rapport à la chambre de pompage (7) et comportant un orifice de sortie (9),
dans lequel un élément d'admission (4) comportant au moins un orifice d'admission (5) est disposé entre la section proximale (2) et la chambre de pompage (7)
**caractérisé en ce que**
l'élément d'admission (4) et la chambre de pompage (7) sont géométriquement espacés l'un de l'autre par un élément d'accouplement (6),
dans lequel l'élément d'accouplement (6) est conçu pour transmettre au moins partiellement à la section proximale (2) une force agissant sur la section distale (3) lors de l'implantation du cathéter (1) dans le corps d'un patient et/ou de son retrait par le fait que l'élément d'admission (4) et/ou la chambre de pompage (7) forment des structures de soutien (16) qui s'étendent dans l'élément d'accouplement (6) et le stabilisent mécaniquement.

2. Cathéter de pompage selon la revendication 1, **caractérisé en ce que** l'élément d'admission (4) est expansible.

3. Cathéter de pompage selon la revendication 1 ou 2,
**caractérisé en ce qu'**une vanne antiretour (14) est disposée au niveau de l'orifice d'admission (5) et/ou dans la zone de l'élément d'accouplement (6).

4. Cathéter de pompage selon l'une des revendications précédentes, **caractérisé en ce que** l'élément d'admission (4) comprend une pluralité d'orifices d'admission (5) ou de vannes d'admission (5').

5. Cathéter de pompage selon la revendication 1, **caractérisé en ce que** l'élément d'accouplement (6) comprend des structures d'accouplement (17) qui viennent en prise par complémentarité de forme et/ou à force avec les structures de soutien (16) de l'élément d'admission (4) et/ou avec les structures de soutien (16) de la chambre de pompage (7) et qui assurent au moins en partie la transmission de force.

6. Cathéter de pompage selon la revendication 5, **caractérisé en ce que** les structures d'accouplement (17) comprennent un matériau non métallique, de préférence un polymère.
